# EUROPEAN PATENT APPLICATION

(11) **EP 2 865 374 A1**
(43) Date of publication of application: **29.04.2015**
(21) Application number: 14190313.8
(22) Date of filing: 24.10.2014
(51) Int. Cl.: A61K 9/48, A61K 9/70, A23L 1/22

(54) **Mixing and extruding method for preparing starch softgel capsules**

(30) Priority: 26.10.2013 CN 201310510570
(71) Applicant: Zhongshan Capsule Starch Material Technology Co., Ltd., Guangdong 528451 (CN)
(72) Inventor: Shuai, Fangwen, 410331 Changsha (CN); Wang, Xiangfeng, 410331 Changsha (CN); Zhang, Jiawei, 410331 Changsha (CN); Zhang, Nuozi, 410331 Changsha (CN)
(74) Representative: Clark, Jane Anne

(57) **Abstract**

Described herein is an extrusion method for preparing starch-based softgel capsules, which, specifically, includes the following 2 steps: 1, co-extruding the starch premix and gel solution to form composite starch film. 2, processing two composite starch films into starch-based softgel capsules by rotary die process.

## Description

### CROSS REFERENCE TO RELATED APPLICATION

This application claims priority from Chinese Patent Application No. 201310510570.0, filed with State Intellectual Property Office, P. R. C. on October 26, 2013, the entire content of which is incorporated herein by reference.

### FIELD OF INVENTION

This invention relates to a preparation method of softgel capsules, more specifically, of non-gelatin softgel capsules.

### BACKGROUND OF THE INVENTION

Softgel capsules are generally used for enclosing the medicines that should not directly contact esophagus and that must be disintegrated in the stomach or intestines. At present, most softgel capsules used for medicines or dietary supplements are made of gelatin extracted from animal skins or the bones, and the extraction process involves acid or alkali treatment, which unavoidably generates animal protein residues. These residues will interact with the enclosed content in the softgel capsules and thus lead to negative consequences such as drug spoilage.

In addition, animal-source gelatin is not acceptable to vegetarians and/or the people with Islamic and Jewish beliefs, and those with allergic constitution should also avoid intake of gelatin products.

As starch is made from rich resource of plants, its price is far lower than that of gelatin. The starch, when modified, achieves better gelation performance and mechanical strength, which makes its characteristics close to that of the gelatin. Since starch-based softgel capsules can overcome the shortcomings of existing gelatin softgel capsules and at the same time has the advantage of low cost, It is worthy of popularization.

By the existing technologies, Pat. No. CN1483393A discloses a starch softgel capsule preparation method, wherein the starch and starch derivatives are dissolved in water and heated to 60-65 °C, and then add plasticizer and put the mixed solution into the heat-insulated trough to prepare the softgel capsule shell using Glue stick method.

Pat. No. CN 103055317A discloses a preparation method of instant plant softgel capsules wherein the raw material is dissolved in water and then made into softgel capsules using softgel machine.

The general practice in the above mentioned methods is to add water to the material to form aqueous solution, which is then input to the softgel capsule machine to form softgel capsules. However, due to the poor water-solubility of starch and other polymers, it is not easy to obtain uniform mixture using water phase---thus affects the quality of the finished product and particularly, leads to the problems like uneven softgel capsule shells and improper encapsulation.

In this invention, the raw material is mixed in a screw extruder. The advantage of this mixing method lies in that the starch

(or starch derivatives) and additives are heated to a relatively higher temperature inside the extruder and melt into a semi-solid form, which is then repeatedly pressed, kneaded, cut, and blended by the screw. The combined effects of these actions can change the molecular chain structure of the starch and lead to solid phase chemical reaction, and thus further improves the mechanical strength of the starch. Additionally, the extruded film has uniform thickness, which overcomes such deficiency as uneven shell thickness and improper encapsulation. It is a great technical progress!

An embodiment of the present invention provides a method of starch-based softgel capsule preparation, aiming to tackle the existing problems of uneven mixture and improper encapsulation in conventional mixing technologies.

The invention includes the following steps:

(a) Preparation of composite starch film: Mix starch, anti-caking agent, and gel solution to form starch-based premix, which is then transferred to the extruder to form composite starch film.

(b) Preparation of starch-based softgel capsules: Process two pieces of composite starch films to starch-based softgel capsules using rotary die process.

### DETAILED DESCRIPTION OF EACH STEP

### Step 1: Preparation of composite starch film

The composite starch film is prepared by mixing the starch and the gel solution through the following steps:

Prepare the gel solution by dissolving gel, water retention agent, emulsifier, and gelatinization agent in water, and mix the starch and anti-caking agent for 5 minutes in the high-speed mixer at the speed of 1500-2000rpm and under the temperature of 60 °C; then slowly add the gel solution into the high-speed mixer while keep stirring for another 5 minutes to get the starch-based premix ready; Feeds the starch-based premix into the double screw-type extruder, through which, the starch-based premix is extruded out as granules. Process the granules into a composite film by single-type extruder.

The raw material is native or modified starch (modified by chemical or physical process), preferably, modified corn starch, potato starch, tapioca starch, wheat starch, mung bean starch, rice starch, or oxidized mung bean starch, of which, mung bean starch should be the priority choice. The suggested carboxyl group content in the modified starch is in the range of 0.2%-0.3%.

The said gel is one or a combination of the substances selected from the group consisting of amylopectin, gellan gum, carrageenan, xanthan gum, and guar gum , among which, carrageenan is the priority choice.

For starch, preferably oxidized mung bean starch, and for gel, preferably carrageenan gel.

The said water retention agent in Step 1 is pharmaceutical glycerin or pharmaceutical grade sorbitol.

The said anti-caking agent mentioned in Step 1 is pharmaceutical stearic acid or fatty glyceride

The said emulsifier in Step 1 is ionic emulsifier, preferably pharmaceutical alkali metal salts sulfate or alkali metal salts sulfonate, such as pharmaceutical sodium dodecyl sulfonate or pharmaceutical sodium dodecyl sulfate.

The said gelatinization agent Step 1 is deionized water.

The parameters of the double screw-type extruder mentioned in Step 1 are as follows: material temperature, 80-170 °C; screw rotation speed, 60-400 rpm; (Optimized speed should be: 90-160°C, 100-150 °C, 100-140 °C, 110-130 °C, 120 °C, 125 °C.)

The parameters of the single screw-type extruder mentioned in Step 1 are as follows: material temperature, 80-165°C; screw rotation speed, 60-300 rpm; (Optimized speed should be: 90-160 °C, 100-150°C, 100-140°C, 110-130°C, 100 °C, 105 °C.).

The composition of the gel solution:

Gel: 15%-25% of the gel solution (by weight)

Water retaining agent: 1%-5% of the gel solution (by weight)

Emulsifier: 0.02-1% of the gel solution (by weight)

Deionized water.

### Step 2: Preparation of starch-based softgel--Rotary die method

Feed two pieces of composite starch films into the two adjoining cylinder moulds respectively; adjust the mould temperature to 40-90 °C and start up the machine. Then, the two cylinder moulds drive the two composite films spinning inward in different direction. The grooves on the mould can be vacuumed to make the composite starch film adhered to the mould form pits, which are then pressed by the mould into softgel capsules with cavities. At the same time of the softgel capsule formation, the softgel capsule content is filled from right above the junction of the two cylinder moulds.

The advantage of this invention is that, the premixed material is thoroughly and uniformly mixed, stirred, kneaded, and blended through the extruder, and meanwhile, the extruder ensures continuous production, and thus reduces the cost.

The starch-based softgel capsule prepared according to this present invention can be used for the preparation of drugs, dietary supplements, and functional foods.

### EXAMPLES

The examples set forth below further explain the content of this present invention and the nature of the product produced by this invention. The following examples are illustrative, and should not be viewed as limiting the scope of the present invention

### Example 1:

Raw materials and their weight ratio:

Gel: carrageenan, 2%;

Starch: corn starch, 75%;

Water retaining agent: pharmaceutical glycerin, 1%;

Anti-caking agent: pharmaceutical stearic acid, 0.2%;

Emulsifier: pharmaceutical sodium dodecyl sulfate, 0.02%;

Gelatinization agent: Deionized water, 21.78%.

The parameters of the double screw-type extruder are as follows

The rotation speed is set to 350 RPM, and the designed temperature for each slider is as follows:
Slide 1: 25 °C
Slide 2-3: 110 °C
Slide 4-6: 145 °C
Slide 7-9: 165 °C
Slide 10-12: 160 °C
Nozzle: 160 °C

The parameters of the single screw-type extruder are as follows

Extrudes at the rotation speed of 300 RPM, and the designed temperature is 120 °C.

Steps to prepare composite films: Prepare the gel solution by dissolving gel, water retention agent, emulsifier, and gelatinization agent in water, and mix the starch and anti-caking agent for 5 minutes in the high-speed mixer at the speed of 1500-2000rpm and under the temperature of 60 °C; Then slowly add the gel solution into the high-speed mixer while keep stirring for another 5 minutes to get the starch-based premix ready; Feeds the starch-based premix into the double screw-type extruder to extrude the premixed material into mixed starch granules, which are then made into composite films made of starch and gel solution by single screw-type extruder. Starch-based softgel capsules preparation: Put the composite film into the rotary die with temperature control device to make starch-based softgel. The mold temperature is set at 60 °C.

### Example 2

Raw materials and their weight ratio:

Gel: Xanthan gum, 2%;

Starch: corn starch, 75%;

Water retaining agent: pharmaceutical glycerin, 3%;

Anti-caking agent: pharmaceutical stearic acid, 0.2%;

Emulsifier: pharmaceutical grade twelve sodium alkyl sulfate, 0.02%;

Gelatinization agent: Deionized water, 19.78%;

Other parameters and processes are the same as those in Example 1.

### Example 3

Raw materials and their weight ratio:

Gel: guar gum, 2%;

Starch: starch, 75%;

Water retaining agent: pharmaceutical grade glycerin, 1%;

Anti-caking agent: pharmaceutical grade stearic acid, 0.5%;

Emulsifier: pharmaceutical grade twelve sodium alkyl sulfate, 0.02%;

Gelatinization agent: Deionized water, 21.48%;

Other parameters and processes are the same as those in Example 1.

### Example 4

### Raw materials and their weight ratio:

### Gel: carrageenan, 2%;

Starch: oxidized mung bean starch 75%, with carboxyl content of 0.1%

### Water retaining agent: pharmaceutical glycerin, 1%;

### Anti-caking agent: pharmaceutical stearic acid, 0.5%;

Emulsifier: Gelatinization agent: pharmaceutical sodium dodecyl sulfate, 0.3%;

Gelatinization: Deionized water, 21.2%;

Other parameters and processes are the same as those in Example 1.

### Example 5

Raw materials and their weight ratio:

Gel: cross-linking cassava starch, 10%;

Starch: wheat starch, 65%;

Water retaining agent: pharmaceutical glycerin, 0%;

Anti-caking agent: pharmaceutical stearic acid, 0%;

Emulsifier: pharmaceutical sodium dodecyl sulfate.

Gelatinization agent: Deionized water, 25%;

Other parameters and processes are the same as Example 1.

### Example 6

Raw materials and their weight ratio:

Gel: carrageenan, 2%;

Starch: oxidized mung bean starch, 80% with carboxyl content of 0.4%

Water retaining agent: pharmaceutical glycerin, 1%;

Anti-caking agent: pharmaceutical stearic acid, 0.2%;

Emulsifier: pharmaceutical sodium dodecyl sulfate, 0.02%.

Gelatinization agent: deionized water. 16.78%.

Other parameters and processes are the same as those in Example 1.

### Example 7:

### Raw materials and their weight ratio:

Gel: carrageenan, 2%;

Starch: oxidized mung bean starch, 80% with carboxyl content of 0.28.

Water retaining agent: pharmaceutical glycerin, 1%;

Anti-caking agent: pharmaceutical stearic acid, 0.2%;

Emulsifier: pharmaceutical sodium dodecyl sulfate, 0.02%;

Gelatinization agent: Deionized water, 16.78%.

Other parameters and processes are the same as those in Example 1.

### Example 8

Raw materials and their weight ratio:

Gel: guar gum, 2%;

Starch: oxidized mung bean starch, 80% with carboxyl content of 0.28.

Water retaining agent: pharmaceutical grade glycerin, 1%;

Anti-caking agent: pharmaceutical stearic acid, 0.2%;

Emulsifier: pharmaceutical sodium dodecyl sulfate, 0.02%;

Gelatinization agent: Deionized water. 16.78%.

Other parameters and processes are the same as Example 1.

### Comparative Example.

Raw materials and their weight ratio:

Gel: carrageenan, 2%;

Starch: cassava starch, 75%;

Water retaining agent: pharmaceutical glycerin, 1%;

Anti-caking agent: pharmaceutical stearic acid, 0.2%;

Emulsifier: pharmaceutical sodium dodecyl sulfate, 0.02%;

Gelatinization agent: Deionized water, 21.78%.

Put the above materials into a sandwich-type pot and stir, and then add the same amount (by weight) of deionized water, stir and steam heat to make it melt. Maintain the temperature for 1 ∼ 2 hours and allow it to stay static until the foams float up. Keep warm and filter to make it paste-like solution for use. Apply conventional method to prepare softgel capsules.

The softgel capsule prepared by the invention is used for encapsulating essential oil. The following result is based on a 6-month long examination on the stability of the softgel capsules within specific time and on the condition of (T = 40± 2 °C, RH = 75%), using essential oil of ligusticum chuanxiong hort as sample material. Use the content of ligustilide as the index to inspect the characters and disintegration time. Disintegration time measurement: follow the method A in the appendix X of Chinese Pharmacopoeia 2010 2nd edition, according to which, the softgel capsuleshould be completely disintegrated within 1 hour; Ligustilide content measurement: apply regular method to measure ligustilide content. Calculate the average value of ligustilide content and disintegration time (sample size: 10 softgel capsules)

**Table 1 Stability Study**

| Examples | Time (unit: month) | Appearance | disintegration time limit | Ligustilide content |
|---|---|---|---|---|
| Examples | 0 | Clean and leakage free | 35 | 100.2% |
| | 1 | Clean and leakage free | 35 | 99.8% |
| | 3 | Clean and leakage free | 37 | 92.5% |
| | 6 | Clean and leakage free | 40 | 83.1% |
| Examples | 0 | Clean and leakage free | 35 | 100.5% |
| | 1 | Clean and leakage free | 36 | 98.3% |
| | 3 | Clean and leakage free | 38 | 93.5% |
| | 6 | Clean and leakage free | 41 | 84.1% |
| Examples | 0 | Clean and leakage free | 34 | 99.9% |
| | 1 | Clean and leakage free | 36 | 97.9% |
| | 3 | Clean and leakage free | 39 | 90.6% |
| | 6 | Clean and leakage free | 42 | 83.9% |
| Examples | 0 | Clean and leakage free | 30 | 100.5% |
| | 1 | Clean and leakage free | 30 | 99.5% |
| | 3 | Clean and leakage free | 32 | 98.9% |
| | 6 | Clean and leakage free | 37 | 90.6% |
| Examples | 0 | Clean and leakage free | 37 | 99.8% |
| | 1 | Clean and leakage free | 38 | 96.9% |
| | 3 | Clean and leakage free | 39 | 90.2% |
| | 6 | Clean and leakage free | 43 | 82.9% |
| Examples | 0 | Clean and leakage free | 29 | 100.1% |
| | 1 | Clean and leakage free | 30 | 99.6% |
| | 3 | Clean and leakage free | 32 | 98.7% |
| | 6 | Clean and leakage free | 37 | 91.2% |
| Examples | 0 | Clean and leakage free | 25 | 100.3% |
| | 1 | Clean and leakage free | 27 | 99.8% |
| | 3 | Clean and leakage free | 30 | 99.0% |
| | 6 | Clean and leakage free | 31 | 98.7% |
| Examples | 0 | Clean and leakage free | 38 | 100.1% |
| | 1 | Clean and leakage free | 39 | 98.1% |
| | 3 | Clean and leakage free | 41 | 93.2% |
| | 6 | Clean and leakage free | 43 | 83.2% |
| Comparative Example | 0 | Clean and leakage free | 52 | 99.9% |
| | 1 | Clean and leakage free | 55 | 92.7% |
| | 3 | Slight leakage on outer wall | 67 | 84.6% |
| | 6 | Serious leakage on outer wall | 76 | 70.3% |

An embodiment provides an extrusion method for preparing starch-based softgel capsules, which, specifically, includes the following 2 steps: 1, co-extruding the starch premix and gel solution to form composite starch film. 2, processing two composite starch films into starch-based softgel capsules by rotary die process.

## Claims

1. A Method for preparing starch-based softgel capsules, comprising:
(1) Preparation of composite starch extrusion film comprising:
mix starch, anti-caking agent, and gel solution to form premixed material; feed into an extruding machine to be processed into a composite starch extrusion film; and
(2) Preparations of starch-based softgel capsules comprising:
process two pieces of composite starch films prepared by step 1 into starch-based softgel capsules by rotary die process.

2. The method according to claim 1, wherein said premixing method of starch and gel solution follows these steps: 1, Dissolve gel, water retention agent, emulsifier, and gelatinization agent in water to form gel solution; 2, Mix the starch and anti-caking agent into a high speed mixer at the speed of 1500-2000rpm; 3, Slowly add the prepared gel solution into the said high speed mixer while keep stirring to form premixed material.

3. The method according to claim 2, wherein the said gel is one or a combination of the substances selected from the group consisting of amylopectin, gellan gum, carrageenan, xanthan gum, and guar gum.

4. The method according to claim 2 or 3, wherein the said water retention agent is polyhydric fatty alcohol; the emulsifier is ionic emulsifier; gelatinization agent is DI water; and anti-caking agent is pharmaceutical stearic acid or glycerol esters of fatty acids.

5. The method according to any preceding claim, wherein the film-forming method of the said composite starch film is to extrude the premixed material into starch granules using a double-screw type extruder, and then make the starch granules into composite starch film using a single-screw extruder.

6. The method according to claim 5, wherein the temperature of the premixed material inside the double-screw extruder is set to 80-170 °C, and the rotation speed is 60-450 rpm.

7. The method according to claim 5, wherein the temperature of the premixed material inside the single-screw extruder is set to 80-165 °C and the rotation speed is 100-380 rpm.

8. The starch-based softgel capsule prepared according to any preceding claim for use in the preparation of drugs, dietary supplements, and functional foods.
